# FASCICULE DE BREVET EUROPEEN

(11) **EP 1 039 878 B1**
(45) Date de publication et mention de la délivrance du brevet: **25.09.2002**
(21) Numéro de dépôt: 98962520.7
(22) Date de dépôt: 18.12.1998
(51) Int. Cl.: A61K 7/48

(54) **UTILISATION DES COMPOSITIONS COSMETIQUES CONTENTANT AU MOINS UNE AUXINE**
VERWENDUNG VON KOSMETISCHEN ZUSAMMENSETZUNGEN, DIE MINDESTENS EIN AUXIN ENTHALTEN
USE OF COSMETIC COMPOSITIONS CONTAINING AT LEAST ONE AUXIN

(30) Priorité: 19.12.1997 FR 9716177
(43) Date de publication de la demande: 04.10.2000
(73) Titulaire: L'OREAL, 75008 Paris (FR)
(72) Inventeur: BRETON, Lionel, F-78000 Versailles (FR); GIRERD-DUGUE, Florence, F-34130 Maugio (FR); RENAULT, Béatrice, F-94410 Saint Maurice (FR)
(74) Mandataire: Galup, Cédric Olivier Nicolas
(86) Numéro de dépôt international: FR9802796
(87) Numéro de publication internationale: WO99032078

(56) Documents cités:
- EP-A- 0 060 553
- EP-A- 0 103 878
- EP-A- 0 451 889
- FR-A- 1 269 573
- P. ROVESTI: "Recherches sur l'action des auxines et des phytohormones en cosmétique" PARFUMERIE MOD., vol. 48, no. 54, 1956, pages 86-91, XP002082741

## Description

L'invention se rapporte à l'utilisation d'au moins une auxine dans une composition cosmétique en tant qu'agent favorisant la synthèse des lipides de la peau.
En particulier, les compositions de l'invention sont destinées à stimuler la synthèse des lipides totaux de la peau particulièrement ceux de l'épiderme.

La peau humaine est constituée de deux compartiments à savoir un compartiment profond, le derme et un compartiment superficiel, l'épiderme.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il contient également des vaisseaux sanguins et des fibres nerveuses.

L'épiderme est en contact avec l'environnement extérieur. Son rôle consiste à protéger l'organisme de la déshydratation et des agressions extérieures, qu'elles soient chimiques, mécaniques, physiques ou infectieuses.
L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.
Les cellules constituant l'épiderme sont délimitées par un domaine lipidique intercellulaire. Au cours de la différenciation, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de sphingolipides.
Ces lipides sont organisés en structures lamellaires spécifiques dont l'intégrité dépend non seulement de la qualité des fractions présentes mais aussi de leur proportion respective. Cette structure lamellaire des lipides du domaine lipidique intercellulaire de l'épiderme est responsable de la fluidité donc de la souplesse de la peau.
Les lipides sont également responsables des propriétés "barrière" de l'épiderme particulièrement du stratum corneum.
Les lipides épidermiques sont synthétisés principalement dans l'épiderme vivant. Ils sont principalement constitués de phospholipides, de sphingolipides, de cholestérol, d'acides gras libres, de triglycérides, d'esters du cholestérol et d'alcanes.
Les phospholipides sont essentiels pour la constitution des membranes cellulaires. Ils jouent un rôle important dans la médiation des signaux extracellulaires et la formation de chaînes aliphatiques libres utilisées pour la production d'énergie. Ils constituent un réservoir d'acides gras libres nécessaires à la constitution des sphingolipides.
Les sphingolipides (ou céramides) sont essentiels pour le maintien de la structure multilamellaire des lipides intercornéocytaires. Ils sont également essentiels pour les échanges en eau et la fonction "barrière" de l'épiderme.
Le cholestérol joue un rôle primordial dans l'hydratation cutanée et dans la fonction "barrière" de l'épiderme.
Les acides gras libres jouent un rôle majeur dans le maintien de la structure lamellaire des lipides du stratum corneum, mais également dans la constitution des membranes cellulaires où ils sont responsables de la fluidité membranaire mais également de processus physiologiques tels que le fonctionnement de récepteurs ou l'activité enzymatique.

On comprend alors le rôle essentiel que jouent les lipides de la peau et l'importance que revêt leur intégrité.

On sait malheureusement que les lipides de la peau, particulièrement de l'épiderme, sont influencés par des facteurs génétiques, le vieillissement, les régimes alimentaires, les saisons, les facteurs environnementaux, les agressions externes et/ou certaines pathologies (scorbut ou pellagre par exemple). Tous ces facteurs ont pour conséquence d'altérer ou encore de modifier la composition des lipides de la peau ou d'en diminuer la quantité, ce qui conduit invariablement à une peau sèche. On sait par exemple que l'absence de la composante lipidique dans un régime a pour conséquence une peau en mauvaise santé. L'absence de lipides conduit à une détérioration générale de la santé et particulièrement à l'apparition d'une peau écailleuse avec une augmentation concomitante de l'augmentation de la perte trans-épidermale en eau.
Les lipides de la peau sont donc essentiels dans le maintien de la "barrière" hydrique de la peau.

On sait aussi que les lipides de l'épiderme ont également une influence sur l'activité de certaines enzymes dans la peau impliquées dans la maturation et dans la desquamation du stratum corneum.
Des variations dans le taux et le type de lipides présents dans le stratum corneum influencent donc la fonction "barrière" du stratum corneum, le contenu en eau et l'état de la peau.

On sait aussi qu'à la ménopause les femmes se plaignent de ce que leur peau tire et qu'elle prend l'aspect d'une "peau sèche", voire de l'apparition d'une xérose. Sans chercher à établir une quelconque théorie sachant que les lipides de la peau jouent un rôle important dans l'hydratation cutanée et que les déficits hormonaux associés à la ménopause s'accompagnent d'un ralentissement général du métabolisme cellulaire, on peut quand même supposer que la sensation de peau qui tire ou de peau sèche que ressent la femme est notamment liée à une diminution de la quantité des lipides totaux de la peau.

On comprend qu'il est donc important de pouvoir stimuler la synthèse des lipides de la peau pour maintenir et/ou restaurer leur intégrité afin de leur permettre de jouer les rôles importants qui leur sont dévolus.

A cet égard la demanderesse a de manière surprenante et inattendue découvert que certaines hormones végétales, ou phytohormones, particulièrement les auxines, présentent la propriété de stimuler la synthèse des lipides, particulièrement des lipides totaux de la peau.

Les auxines sont des phytohormones, c'est à dire des hormones végétales au même titre que les gibberellines, les cytokinines, l'éthylène ou l'acide abscissique.

Les hormones végétales sont des messagers chimiques servant d'intermédiaires dans les communications intercellulaires chez les plantes supérieures. Ce sont en général des molécules relativement petites qui agissent en très petites quantités. Les hormones végétales interviennent dans la régulation des processus de développement des végétaux.

Les auxines sont des hormones végétales intervenant dans la régulation de l'élongation des cellules végétales et dans la croissance des plantes en réponse à un stimulus unidirectionnel, phénomène connu sous le nom de tropisme (voir "Plant propagation by tissue culture", George E. F. et Sherrington P. D., 1984, Exegetics Limited ou "Dictionnary of natural products" de Chapman et Hall, 1997).

Dans l'art antérieur, les auxines sont connues pour être utilisées comme régulateur du flux calcique de part et d'autres des membranes cellulaires (EP 240 257), ou comme entrant dans des compositions antitumorale (FR 2 597 339), pour le traitement des blessures de la surface de la peau (EP 60 553) ou encore pour le traitement des brûlures ou ulcères (EP 103 878).
A la connaissance de la demanderesse il n'a jamais été décrit dans l'état de la technique l'utilisation d'au moins une auxine pour stimuler la synthèse des lipides totaux de la peau.

L'invention a donc pour objet l'utilisation dans une composition cosmétique d'une quantité efficace d'au moins une auxine, l'auxine ou la composition étant destinée à stimuler la synthèse des lipides totaux de la peau.

Le terme auxine est utilisé pour décrire des substances naturelles et/ou synthétiques qui stimulent l'élongation des coléoptiles et des tiges des végétaux.
Ainsi, selon l'invention, les auxines peuvent être d'origine naturelle ou synthétique.
Par auxine naturelle, on entend toute auxine, ou toute préparation la contenant, présente à l'état naturel dans au moins un végétal.
Par auxine synthétique, on entend tout composé ayant une activité similaire ou identique aux auxines naturelles, mais qui a été obtenu par synthèse chimique ou par biotechnologie.
Ainsi, par la suite dans le texte le terme auxine s'entend comme désignant une auxine naturelle ou synthétique purifiée ou toute préparation contenant une auxine naturelle ou synthétique.

Les auxines utilisables selon l'invention sont celles décrites dans les deux ouvrages de référence cités plus haut dans le texte. Parmi celles-ci on peut citer à titre d'exemple l'acide 3-indolacétique (IAA), l'acide 4-chloroindole-3-acétique (4-CI-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'idole acétaldéhyde et l'indole acétonitrile.

Préférentiellement, selon l'invention on utilise l'acide β-naphtoxyacétique.

Bien entendu, il est possible d'utiliser selon l'invention les auxines seules ou en mélange.

Particulièrement l'auxine ou la composition la contenant sont utilisées selon l'invention en application topique sur la peau.

On a vu précédemment que les lipides sont impliqués entre autres dans la fonction "barrière" de la peau, dans l'hydratation cutanée ou encore dans la souplesse de la peau. On a vu également que la ménopause induit des effets cutanés particulièrement sur les lipides de la peau.

Ainsi, un des aspects de l'invention est donc de proposer l'utilisation dans une composition cosmétique d'une quantité efficace d'au moins une auxine, l'auxine ou la composition étant destinée à renforcer la fonction "barrière" de la peau.

Selon un autre aspect, l'invention a pour objet l'utilisation dans une composition cosmétique d'une quantité efficace d'au moins une auxine, l'auxine ou la composition étant destinée à favoriser l'hydratation cutanée.

Selon encore un autre aspect l'invention a pour objet l'utilisation dans une composition cosmétique d'une quantité efficace d'au moins une auxine, l'auxine ou la composition étant destinée à renforcer la souplesse de la peau.

Selon encore un autre aspect l'invention a pour objet l'utilisation dans une composition cosmétique d'une quantité efficace d'au moins une auxine, l'auxine ou la composition étant destinée à combattre les effets cutanés de la ménopause, plus particulièrement les effets cutanés de la ménopause liés à la diminution de la quantité des lipides de la peau.

La quantité d'auxine utilisable selon l'invention dépend bien évidemment de l'effet recherché et doit être en une quantité efficace pour stimuler la synthèse des lipides de la peau.

A titre d'exemple la quantité d'auxine utilisable selon l'invention peut aller par exemple de 10⁻⁶% à 10% et de préférence de 10⁻³% à 5% du poids total de la composition.

La composition utilisée selon l'invention comprend bien entendu un support cosmétiquement acceptable et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

De façon connue, la composition de l'invention peut contenir également les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétales (huile d'abricot, huile de tournesol), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Il est également possible d'utiliser en association avec l'auxine utilisée selon l'invention des composés choisis parmi
- les agents antibactériens tels que les macrolides, les pyranosides et les tétracyclines, et notamment l'érythromycine ;
- les agents antagonistes de calcium, comme le verapamil et le diltiazem ;
- des capteurs de radicaux OH, tels que le diméthylsulfoxyde ;
- des extraits de végétaux tels que ceux d'lridacés ou de soja, extraits pouvant alors contenir ou non des isoflavones ;
- des extraits de micro-organismes dont en particulier des extraits bactériens comme ceux de bactéries filamenteuses non photosynthétiques ;

A la liste ci-dessus, d'autres composés peuvent également être rajoutés, à savoir par exemple les ouvreurs de canaux potassiques tels que le diazoxyde et le minoxidil,, la spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétraénoïque et eicosatriénoïque ou leurs esters et amides, la vitamine D et ses dérivés.

Selon l'invention, on peut, entre autres, associer au moins une auxine à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle
- des substances telles que les antagonistes de substance P, de CGRP ou de bradykinine ou les inhibiteurs de NO synthase, composés décrits comme étant actifs dans le traitement des peaux sensibles et comme présentant des effets anti-irritants, en particulier vis-à-vis de composés irritants éventuellement présents dans les compositions.

Ainsi, un autre objet de l'invention concerne une composition comprenant une quantité efficace d'au moins une auxine et au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques, les agents modulant la différenciation et/ou la prolifération et/ou la pigmentation cutanée, les antagonistes de substance P, de CGRP ou de bradykinine ou les inhibiteurs de NO synthase.

Comme actifs, on peut utiliser notamment les hydratants tels que les polyols (par exemple la glycérine), les vitamines (par exemple le D-panthénol), les agents anti-inflammatoires, les agents apaisants (allantoïne, eau de bleuet), les filtres UVA et UVB, les agents matifiants (par exemple les polydiméthylorganosiloxanes partiellement réticulés vendus sous le nom KSG® par Shin Etsu), et leurs mélanges.

On peut aussi ajouter des actifs antirides, et notamment des produits tenseurs tels que les protéines végétales et leurs hydrolysats, en particulier l'extrait de protéines de soja vendue sous le nom d'Eleseryl® par la société LSN ou le dérivé d'avoine vendu sous la dénomination Reductine® par la société Silab.

Bien entendu, les auxines peuvent être utilisées dans la préparation de compositions cosmétiques et/ou pharmaceutiques, particulièrement dermatologiques, destinées à stimuler la synthèse des lipides,

D'autres caractéristiques et avantages de l'invention ressortiront mieux des exemples qui suivent donnés à titre illustratif et non limitatif. Dans ce qui suit ou ce qui précède, les proportions sont données en pourcentage pondéral, sauf indications contraires.

### Exemple 1 : Etude de l'effet de l'acide β-naphtoxyacétique sur la synthèse des lipides totaux de la peau.

L'étude est faite par mesure de l'incorporation d'acétate marqué au carbone 14 dans des modèles d'épiderme humain reconstruit vendu par la société Skinethic, dont la composition en lipides totaux est proche de celle de l'épiderme humain normal.
Les cultures d'épiderme humain reconstruit sont effectuées selon recommandation du fournisseur.

L'acide β-naphtoxyacétique, aux concentrations de 10⁻⁵, 10⁻⁶, et 10⁻⁷ M est mis en contact pendant 72 heures avec des épidermes humains reconstruits après 14 jours de culture de ces derniers selon les conditions recommandées par le fournisseur. Le marquage à l'acétate ¹⁴C, ([2-¹⁴C]-acétate de sodium vendu par Amersham, 59 mCi/mmol) est effectué 24 heures après mise en contact du produit à tester avec la culture, soit pendant les 48 dernières heures de culture, à raison de 0,5µCi d'acétate ¹⁴C par culture.

En fin de culture, après lavage des épidermes en tampon phosphate (PBS), les épidermes sont dissociés et les cellules lysées par de l'acide perchlorique à 0,5 M sur de la glace. Les lysats subissent alors une extraction par un mélange méthanol/chloroforme (2:1), une centrifugation et de nouveau une extraction sur les culots obtenus dans les mêmes conditions que précédement. Les lipides sont séparés par addition de PBS et de chloroforme (technique de Blight et Byer). La phase organique contenant les lipides est prélevée et la radioactivité incorporée dans cette phase est déterminée par scintillation liquide. La phase organique est ensuite séchée sous flux d'azote. Une chromatographie en couche mince sur plaque sur plaques (Merck K60) en utilisant comme éluant un mélange chloroforme/méthanol/eau (50:18:2,6) pour séparer les phospholipides ou un mélange hexane/éther/acide acétique (15:5,6:0,19) pour séparer les lipides neutres.
Les plaques sont ensuite autoradiographiées pendant 24 heures et les chromatogrammes sont analysé par densitométrie à l'aide du logiciel One-D-Scan de la société Scanalytics.

Les résultats sont évalués par rapport à un témoin constitué par des cellules n'ayant pas été traitées à l'acide β-naphtoxyacétique.

Un témoin positif (trifluopérazine à 10⁻⁴M) connu pour stimuler la synthèse des lipides et un témoin négatif (l'acide rétinoïque à 10⁻⁶ M) sont introduits dans le test comme référence.

Les résultats de ce test exprimés en pourcentage de stimulation sont présentés dans le tableau suivant.

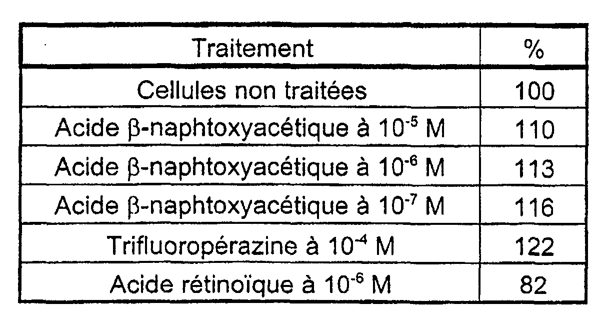

Ces résultats montrent que l'acide β-naphtoxyacétique stimule de façon significative l'incorporation d'acétate montrant un effet sur la synthèse des lipides.

### Exemple 2 : Exemples de compositions selon l'invention. Ces compositions sont obtenues par les techniques habituelles couramment utilisées en cosmétique ou en pharmacie.

| Composition 1 : crème de soin | |
|---|---|
| Cire d'abeille | 1,5 % |
| Huile d'amandes d'abricot | 13,0 % |
| Parfum | 0,4 % |
| Acide β-naphtoxyacétique | 0,01 % |
| Xanthane | 0,5 % |
| Cyclopentadiméthyisiloxane | 5,0 % |
| Eau déminéralisée stérilisée | 69,29 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |
| Conservateurs | 0,3 % |

| Composition 2 : huile corporelle | |
|---|---|
| Huile de vaseline | 47,99 % |
| Huile d'amandes d'abricot | 6,0 % |
| Parfum | 1,0 % |
| Acide β-naphtoxyacétique | 0,01 % |
| Cyclopenta diméthylsiloxane | 45,0 % |

| Composition 3 : lait démaquillant | |
|---|---|
| Palmitate d'éthyl-2 hexyle | 10,5 % |
| Fraction liquide de beurre de karité | 16,5 % |
| Conservateurs | 0,3 % |
| Parfum | 0,15 % |
| Acide β-naphtoxyacétique | 0,01 % |
| Hydroxyde de sodium | 0,04 % |
| Polymère carboxyvinylique | 0,2 % |
| Eau déminéralisée stérilisée | 69,8 % |
| Mélange de cétylstéarylglucoside et d'alcools cétylique et stéarylique | 2,5 % |

| Composition 4 : crème de soin | |
|---|---|
| Cire d'abeille | 1,5 % |
| Huile d'amandes d'abricot | 13,0 % |
| Conservateurs | 0,3 % |
| Parfum | 0,4 % |
| Triéthanolamine | 0,17 % |
| Acide β-naphtoxyacétique | 0,01 % |
| Acide 2,4-dichlorophenoxyacetique | 0,01 % |
| Xanthane | 0,5 % |
| Cyclopenta diméthylsiloxane | 5,0 % |
| Eau déminéralisée stérilisée | 69,28 % |
| Mono-di-palmitostéarate de sucrose | 3,0 % |
| Sesquistéarate de méthylglucose | 3,0 % |

## Revendications

1. Utilisation d'une auxine ou d'une composition cosmétique contenant une quantité efficace d'au moins une auxine, pour stimuler la synthèse des lipides de la peau.

2. Utilisation selon la revendication 1, pour renforcer la fonction "barrière" de la peau.

3. Utilisation selon la revendication 1, pour favoriser l'hydratation cutanée.

4. Utilisation selon la revendication 1, pour renforcer la souplesse de la peau.

5. Utilisation selon la revendication 1, pour combattre les effets cutanés de la ménopause.

6. Utilisation selon l'une quelconque des revendications précédentes, **caractérisée par le fait que** la composition est utilisée en application topique sur la peau.

7. Utilisation selon l'une quelconque des revendications précédentes, **caractérisée par le fait que** l'auxine est choisie parmi l'acide indolacétique (IAA), l'acide 4-chloroindole-3-acétique (4-Cl-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'indole acétaldéhyde et l'indole acétonitrile.

8. Utilisation selon la revendication précédente, **caractérisée par le fait que** l'auxine est l'acide β-naphtoxyacétique.

9. Utilisation selon l'une quelconque des revendications précédentes, **caractérisée par le fait que** l'auxine est présente en une quantité allant de 10⁻⁶% à 10% du poids total de la composition.

10. Utilisation selon la revendication précédente, **caractérisée par le fait que** l'auxine est présente en une quantité allant de 10⁻⁵% à 5% du poids total de la composition.

## Claims

1. Use of an auxin or of a cosmetic composition containing an effective amount of at least one auxin, to stimulate skin lipid synthesis.

2. Use according to Claim 1, to reinforce the "barrier" function of the skin.

3. Use according to Claim 1, to promote moisturization of the skin.

4. Use according to Claim 1, to reinforce the suppleness of the skin.

5. Use according to Claim 1, to combat the effects of the menopause on the skin.

6. Use according to any one of the preceding claims, **characterized in that** the composition is used in topical application on the skin.

7. Use according to any one of the preceding claims, **characterized in that** the auxin is chosen from indolacetic acid (IAA), 4-chloro-3-indoleacetic acid (4-Cl-IAA), phenylacetic acid (PAA), 3-indolebutyric acid (IBA), 2,4-dichlorophenoxyacetic acid (2,4-D), α-naphthaleneacetic acid (α-NAA), β-naphthoxyacetic acid, indolethanol, indolacetaldehyde and indolacetonitrile.

8. Use according to the preceding claim, **characterized in that** the auxin is β-naphthoxyacetic acid.

9. Use according to any one of the preceding claims, **characterized in that** the auxin is present in an amount ranging from 10⁻⁶% to 10% of the total weight of the composition.

10. Use according to the preceding claim, **characterized in that** the auxin is present in an amount ranging from 10⁻⁵% to 5% of the total weight of the composition.

## Patentansprüche

1. Verwendung eines Auxins oder einer kosmetischen Zusammensetzung, die eine wirksame Menge mindestens eines Auxins enthält, zur Stimulation der Synthese von Lipiden der Haut.

2. Verwendung nach Anspruch 1 zur Verstärkung der "Barriere"-Funktion der Haut.

3. Verwendung nach Anspruch 1 zur Förderung der Hydratisierung der Haut.

4. Verwendung nach Anspruch 1 zur Verbesserung der Geschmeidigkeit der Haut.

5. Verwendung nach Anspruch 1 zur Bekämpfung der Auswirkungen der Menopause auf die Haut.

6. Verwendung nach einem der vorhergehenden Ansprüche, **dadurch gekennzeichnet, dass** die Zusammensetzung topisch auf die Haut angewandt wird.

7. Verwendung nach einem der vorhergehenden Ansprüche, **dadurch gekennzeichnet, dass** das Auxin unter Indolylessigsäure (IES), (4-Chlor-3-indolyl)essigsäure (4-Cl-IES), Phenylessigsäure (PES), 3-Indolylbuttersäure (IBS), (2,4-Dichlorphenoxy)-essigsäure (2,4-D), α-Naphthalinessigsäure (α-NES), β-Naphthyloxyessigsäure, Indolylethanol, Indolylacetaldehyd und Indolylacetonitril ausgewählt ist.

8. Verwendung nach dem vorhergehenden Anspruch, **dadurch gekennzeichnet, dass** es sich bei dem Auxin um β-Naphthyloxyessigsäure handelt.

9. Verwendung nach einem der vorhergehenden Ansprüche, **dadurch gekennzeichnet, dass** das Auxin in einem Mengenanteil von 10⁻⁶ bis 10 % des Gesamtgewichts der Zusammensetzung vorliegt.

10. Verwendung nach dem vorhergehenden Anspruch, **dadurch gekennzeichnet, dass** das Auxin in einem Mengenanteil von 10⁻⁵ bis 5 % des Gesamtgewichts der Zusammensetzung vorliegt.
